# EUROPÄISCHE PATENTSCHRIFT

(11) **EP 0 442 330 B1**
(45) Veröffentlichungstag und Bekanntmachung des Hinweises auf die Patenterteilung: **25.10.1995**
(21) Anmeldenummer: 91101317.5
(22) Anmeldetag: 01.02.1991
(51) Int. Cl.: A61F 2/38

(54) **Kniegelenk-Endoprothese**
Kneejoint endroprosthesis
Endoprothèse pour l'articulation du genou

(30) Priorität: 16.02.1990 DE 4004787; 23.03.1990 DE 4009360
(43) Veröffentlichungstag der Anmeldung: 21.08.1991
(73) Patentinhaber: CERASIV GmbH INNOVATIVES KERAMIK-ENGINEERING, D-73207 Plochingen (DE)
(72) Erfinder: Stock, Dietrich, Prof. Dr., W-3300 Braunschweig (DE); Hund, Walter, W-7602 Oberkirch-Stadelhofen (DE); Vizethum, Freimut, Dr.-Ing., W-6830 Schwetzingen (DE)
(74) Vertreter: Klose, Hans, Dipl.-Phys.

(56) Entgegenhaltungen:
- US-A- 4 016 606
- US-A- 4 085 466
- US-A- 4 586 933

## Beschreibung

Die Erfindung bezieht sich auf eine Kniegelenk-Endoprothese gemäß den im Oberbegriff des Patentanspruchs 1 angegebenen Merkmalen.

Aus der DE 25 50 704 C2 ist eine derartige Kniegelenk-Endoprothese bekannt, in welcher zwei künstliche Meniskuselemente zwischen den sphärisch gekrümmten Lagerflächen des Femurteils und den im wesentlichen ebenen Lagerflächen des Tibiateils angeordnet sind. Die gekrümmten Lagerflächen des Femurteils und der Meniskuselemente erfordern einen hohen Fertigungsaufwand, wobei infolge von Fertigungstoleranzen hohe Flächenpressungen in der Praxis kaum zu vermeiden sind. Um insoweit einen gewissen Ausgleich zu ermöglichen, besteht das Meniskuselement aus einem nachgiebigen Kunststoff. Geringfügige Abweichungen von der Geometrie der Lagerflächen und die hieraus resultierenden Flächenpressungen und Spitzenbelastungen können zu vergleichsweise schneller Abnutzung und zu Beschädigungen führen, wodurch die Lebensdauer des Kniegelenks wesentlich verringert wird. Kunststoffe neigen bekanntlich bei Druckbelastung zu bleibenden Deformationen und die ursprünglich komplementären Lagerflächen verändern ihre Geometrie. Die Ausbildung der Menizkuselemente aus einem nachgiebigen Kunststoff, wie beispielsweise Polyethylen, führt in der Praxis schon nach geraumer Zeit zu bleibenden Verformungen des Meniskuselements, wodurch die ursprünglich vorhandene komplementäre Ausgestaltung der Lagerflächen von Femurteil und Meniskuselement nicht mehr gewährleistet werden kann und insgesamt die Funktionsfähigkeit der Endodprothese in Frage gestellt wird. Das Meniskuselement kann auf der ebenen Lagerfläche des Tibiateils nicht nur lineare Bewegungen durchführen, sondern es kann um die zur ebenen Lagerfläche des Tibiateils orthogonale Achse drehen, welche durch den Mittelpunkt der sphärisch gekrümmten Lagerfläche des Femurteils verläuft. Es sind daher zusätzliche Anschläge vorhanden, um den Drehwinkelbereich des Femurteils vorzugeben. Insoweit besteht die Gefahr, daß durch linienförmige oder gar punktförmige Anlage hohe Flächenpressungen, nachteilig hohe Spitzenbelastungen und Beschädigungen sich ergeben.

Aus der DE 35 28 204 A1 ist eine Kniegelenk-Endoprothese bekannt, bei welcher das Femurteil und das Tibiateil jeweils ein Führungsorgan aufweisen. Diese Führungsorgane sind vorzugsweise als eine Nut und eine Nase ausgebildet, welche miteinander zusammenwirken, um Beugungstellungen in der sagitalen Ebene zu ermöglichen. Die Führungsorgane sind derart ausgebildet, daß eine gegenseitige Drehung zwischen Femurteil und Tibiateil ermöglicht wird, um so einen zusätzlichen Freiheitsgrad entsprechend dem natürlichen Kniegelenk zu erhalten. Auch bei dieser KniegelenkEndoprothese sind die Lagerflächen des Femurteils als sphärisch gekrümmte Flächen ausgebildet, welche wiederum in entsprechend gekrümmten Flächen des Tibiateils gelagert sind. Ein zusätzliches Meniskuselement ist hingegen nicht vorgesehen, sodaß die Bewegungsabläufe nur in grober Näherung einem natürlichen Kniegelenk entsprechen.

Ferner ist aus der US-PS 42 24 696 eine Endoprothese bekannt deren Tibiateil einer gekrümmte Lagerfläche für das aus Kunststoff bestehende Meniskuselement aufweist, wobei der Mittelpunkt dieser gekrümmten Lagerfläche im Bereich des Femurteils liegt. Die Lagerflächen des Femurteils und des Tibiateils liegen im wesentlichen koaxial zueinander und sind näherungsweise entsprechend den Oberflächengestaltungen der natürlichen Gelenkteile gekrümmt ausgebildet. Es ist ein einziges Meniskuselement aus Kunststoff vorhanden, dessen konvex gekrümmte Lagerfläche entsprechend der konkav gekrümmten Lagerfläche des einzigen Tibiateils anliegt. Der Herstellungsaufwand einer derartigen Kniegelenk-Endoprothese ist nicht unerheblich und hohe Flächenpressungen aufgrund linienartiger Berührungen sind in der Praxis nicht auszuschließen.

Der Erfindung liegt daher die Aufgabe zugrunde, die Endoprothese der eingangs genannten Art dahingehend weiterzubilden, daß die physiologische Kniegelenkfunktionen bei Bewegung und Belastung erfüllt werden. Die Kräfte sollen auf möglichst große Flächen übertragbar sein und örtliche Belastungsspitzen, insbesondere infolge von Linien- oder Punktberührung, sollen vermieden werden. Die Endoprothese soll kleine Abmessungen aufweisen. Ferner sollen gute tribologische Eigenschaften hinsichtlich der gegeneinander bewegbaren Teile sowie eine hohe statische Festigkeit der kraftübertragenden Teile gewährleistet sein. Darüber hinaus sollen Reibungskräfte zwischen den gegeneinander bewegbaren Bauteilen minimiert werden.

Die Lösung dieser Aufgabe erfolgt gemäß den im kennzeichnenden Teil des Patentanspruchs 1 angegebenen Merkmalen.

Die vorgeschlagene Kniegelenk-Endoprothese gewährleistet bei zuverlässiger und kompakter Konstruktion eine optimale Realisierung der physiologischen Kinematik und Statik. Die Lagerfläche des Femurteils ist kugelförmig ausgebildet, während die Lagerfläche des Tibiateils zylindrisch ausgebildet ist, wobei die zugeordneten Lagerflächen des Meniskuselements entsprechend kugelförmig bzw. zylindrisch ausgebildet sind. Im Gegensatz zu den körpereigenen Kondylen und deren evolventenartig gekrümmten Lagerflächen weisen die beiden Femurteile kugelförmige Lagerflächen auf, welche ebenso wie die korrespondierenden konkav kugelförmigen Lagerflächen der Meniskuselemente mit hoher Präzision gefertigt werden können. Entsprechendes gilt auch für die koaxialen, zylindrischen Lagerflächen der Tibiateile und der Meniskuselemente. Die Zylinderachsen der Tibiateile liegen entsprechend der Beugeachse im wesentlichen parallel zu einem Tibiaplateau, auf welchem die Tibiateile fest angeordnet sind. Die Drehachsen der zylindrisch, konvex ausgebildeten Lagerflächen der Tibiateile liegen im Bereich der Tibia und es wird hierdurch eine Zwangsführung des Meniskuselements auch der Zylinderfläche gewährleistet und die eingangs erläuterte Drehung zuverlässig verhindert. Anschläge zur Unterbindung einer unzulässigen Drehung der bikonkaven Meniskuselemente um eine durch das Femurteil verlaufende Hochachse sind nicht erforderlich. Durch die Kombination einerseits von kugelförmigen Lagerflächen des Femurteils und andererseits zylindrischen Lagerflächen des Tibiateils erfolgt in zweckmäßiger Weise eine Flächensteuerung und eine Zwangsführung des Meniskuselements auf der Zylinderfläche. In Abhängigkeit von der jeweiligen Beugestellung ist eine definierte Ausrichtung des Meniskuselements bezüglich des Tibiateils sowie des Femurteils gewährleistet und zusätzliche Anschläge, Begrenzungen und dergleichen sind daher nicht erforderlich.

Die Achsen der beiden Zylinderflächen des Tibiateils liegen zweckmäßig in einer gemeinsamen Ebene, wobei diese gemeinsame Ebene in der gestreckten Stellung von Tibiateil und Femurteil im wesentlichen auch durch die Mittelpunkte der beiden kugelförmigen Lagerflächen der Femurteile verläuft. Die beiden Meniskuselemente sind bikonkav ausgebildet, wobei die dem Femurteil zugeordnete Lagerfläche kugelförmig und die dem Tibiateil zugeordnete Lagerfläche zylindrisch ausgebildet ist. Das Meniskuselement kann in besonders zweckmäßiger Weise aus einem harten, deformationsfreien Werkstoff, insbesondere Oxydkeramik, gefertigt sein, wodurch eine lange Lebensdauer der Kniegelenk-Endoprothese von vielen Jahren gewährleistet wird. Dies steht im Gegensatz zu den vorbekannten Kniegelenk-Endoprothesen, deren Meniskuselemente aus einem anderen, und zwar elastischeren bzw. weicheren Kunststoff bestanden. Die Meniskuselemente bestehen in zweckmäßiger Weise aus dem gleichen Werkstoff wie die Femurteile und Tibiateile, wobei die in der Implantologie bewährten biokompatiblen Werkstoffe, und zwar vor allem Keramik, zum Einsatz gelangen. Wie bei dem natürlichen Kniegelenk erfolgt in der gestreckten Stellung des Tibiateils bezüglich des Femurteils eine Arretierung bezüglich Drehung, zumal die beiden Mittelpunkte der kugelförmigen Lagerflächen der Femurteile im wesentlichen in der gleichen Ebene liegen wie die Zylinderachsen der Lagerflächen der Tibiateile. Beim Beugen erfolgt eine Verschiebung der genannten Mittelpunkte nach dorsal und eine Drehung wird nunmehr möglich. Die Meniskuselemente sind bezüglich des Tibiateils nur in zwei Raumrichtungen bewegbar und zwar rotatorisch um die Zylinderachse und translatorisch parallel zur Zylinderachse. Die Zylinderfläche verhindert hierbei aber eine Drehung um eine zum Tibiaplateau orthogonale Hochachse. Auch beim erneuten Strecken erfolgt eine Zwangsführung des Meniskuselements auf der zylindrisch konvexen Lagerfläche des Tibiateils und das Meniskuselement nimmt in der gestreckten Position aufgrund der Zwangsführung entlang der konvex gekrümmten Lagerfläche des Tibiateils wieder die ursprüngliche Position ein.

Funktionsverbessernd können die tibialen Lagerflächen im Rahmen dieser Erfindung auch ein- oder beidseitig gegen das Tibiaplateau gekippt sein, um die Führungsfunktion und die biologische Adaption zu verbessern. Die beiden auf dem Tibiaplateau angeordneten Tibiateile können in ihren Zylinderachsen bezüglich des Tibiaplateaus in der gleichen Richtung oder auch gegensinnig geneigt sein, wobei den Erfordernissen entsprechend die Neigungswinkel vorgebbar sind. Der bzw. die Neigungswinkel können im Bereich zwischen 0 bis ± 45 Grad liegen, wobei sich ein Bereich zwischen 0 bis ± 20 Grad als zweckmäßig erwiesen hat. Es ist wesentlich, daß das Meniskuselement radial und/oder axial bezüglich der zylindrischen Lagerfläche des Tibiateils bewegbar ist. Femurteil, Meniskuselement und Tibiateil liegen mit ihren Lagerflächen in jeder Drehwinkellage von Femurteil und Tibiateil großflächig aneinander, wobei Linienberührungen und Spitzenbelastungen zuverlässig vermieden werden. Das Meniskuselement kann in besonders zweckmäßiger Weise aus dem gleichen Material wie Femurteil und Tibiateil bestehen und insgesamt wird eine hohe Lebensdauer und Funktionssicherheit erreicht. Eine seitliche Führung des Meniskuselements bezüglich des Tibiateils in Richtung der Zylinderachse kann zweckmäßig entfallen, wodurch Ausgleichsbewegungen ermöglicht werden. Durch den intakten Bewegungsapparat mit Bändern und Muskeln werden Femurteil, Meniskuselement und Tibiateil in anatomisch optimaler Weise zusammengehalten, wobei ein dauerhafter Flächenkontakt gewährleistet wird und Linienberührungen und Überbelastungen zuverlässig vermieden werden. Durch die Gestaltung der Gelenkflächen von Tibia und Femur ist darüber hinaus die freie, gewebeschonende Beweglichkeit des Gelenks entsprechend den anatomischen Anforderungen gegeben.

Weiterbildungen und Besonderheiten der erfindungsgemäßen Kniegelenk-Endoprothese werden nachfolgend anhand der Zeichnung näher erläutert. Es zeigen:
- Fig. 1: eine schematische Ansicht von lateral,
- Fig. 2: eine schematische Ansicht von ventral,
- Fig. 3: vergrößert eine Ansicht ähnlich Fig. 1, und zwar für Beugung und Streckung,
- Fig. 4: eine Ansicht in Blickrichtung IV, gemäß Fig. 3,
- Fig. 5: eine Ansicht ähnlich Fig. 3, zur Erläuterung der Lage der Zylinderachse.

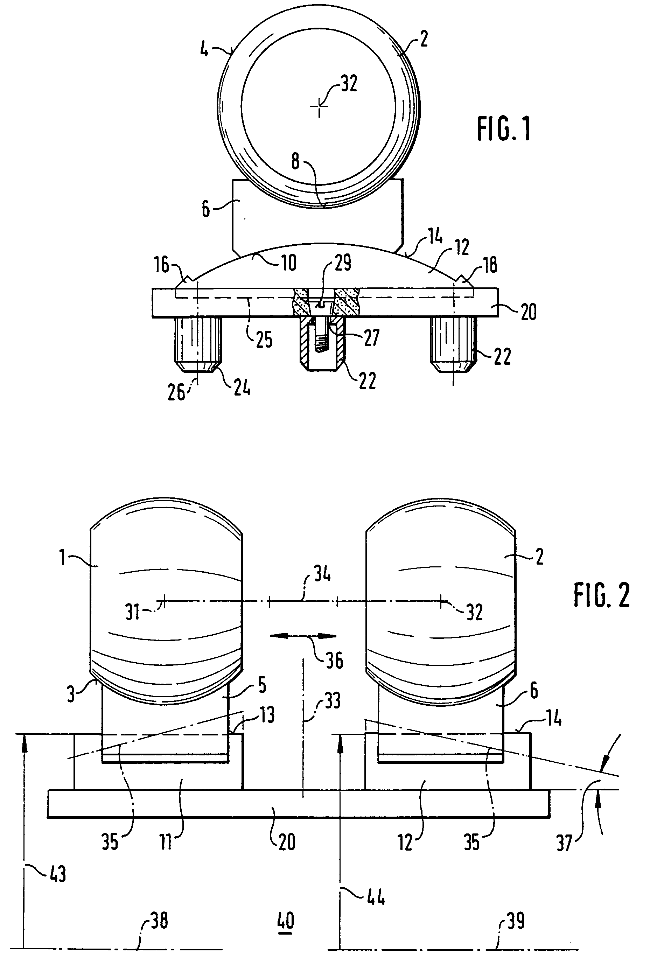

Fig. 1 zeigt von lateral schematisch die Endoprothese mit einem Femurteil 2, dessen Lagerfläche 4 kugelförmig ausgebildet ist. Ein Meniskuselement 6 weist eine gleichfalls kugelförmige erste Lagerfläche 8 auf. Diese erste Lagerfläche 8 liegt konzentrisch zur Lagerfläche 4 des Femurteiles 2, und zwar mit übereinstimmendem Radius. Ferner weist das bikonkave Meniskuselement 6 eine zweite, allerdings zylindrische Lagerfläche 10 auf.

Ferner ist ein Tibiateil 12 mit einer zylindrischen äußeren Lagerfläche 14 vorhanden, auf welcher die koaxiale Lagerfläche 10 des Meniskuselements 6 aufliegt. Wie nachfolgend noch zu erläutern ist, wird beim Beugen das Meniskuselement 6 entlang der zylindrischen Lagerfläche 14 bewegt, wobei zur Begrenzung dieser Bewegung zweckmäßig Begrenzungsteile 16, 18 auf dem Tibiateil 12 angeordnet sind. Das Tibiateil 12 ist mit einem Tibiaplateau 20 fest verbunden. Tibiateil 12 und Tibiaplateau 20 können gegebenenfalls auch einstückig ausgebildet sein. An der Unterseite des Tibiaplateaus 20 sind zylindrische Ansätze 22 mit Schneidspitzen 24 vorgesehen, welche eine zuverlässige Verankerung im Knochen ermöglichen, und zwar mittels hier nicht weiter dargestellten Befestigungsschrauben, welche lediglich durch die strichpunktierten Linien 26 angedeutet sind.

Durch die gestrichelten Linien 25 ist angedeutet, daß das Tibiateil 12 gegebenenfalls ein separates Bauteil darstellt, welches in einer entsprechenden Vertiefung des Tibiaplateaus 20 angeordnet und dort befestigt ist. Der rohrförmige Ansatz 22 weist innen eine konisch selbsthemmende Bohrung 27 für eine Befestigungsschraube 29 auf. Die Befestigungsschraube 29 ist mittels Sicherungselementen gegen Lösen im Tibiaplateau 20 gesichert und kann bedarfsweise wieder gelöst werden, um das Tibiaplateau 20 von der Tibia zu entfernen. In den Ansätzen können beispielsweise zweckmäßig Gewinde für Gewindebolzen vorgesehen sein, um die Befestigungsschrauben gegen Lösen zu sichern.

Fig. 2 zeigt von ventral die Kniegelenk-Endoprothese mit zwei Femurteilen 1, 2 entsprechend den natürlichen Kondylen. In Verbindung mit Fig. 1 ist die kugelförmige Ausgestaltung der Lagerflächen der beiden Femurteile 1, 2 mit den beiden Mittelpunkten 31, 32 und den kugelförmigen Lagerflächen 3, 4 gut zu ersehen. Die Radien der Femurteile 1, 2 werden zweckmäßig gleich groß ausgebildet. Darüber hinaus besteht aber grundsätzlich auch die Möglichkeit die beiden Radien unterschiedlich groß auszubilden. Voraussetzung hierbei ist, daß die Verbindungslinie 34 der beiden Mittelpunkte 31, 32 parallel zu den Zylinderachsen 38, 39 der Lagerflächen der Tibiateile 11, 12 liegt. Die Zylinderachsen 38, 39 liegen in einer gemeinsamen Ebene 40. In der gestreckten Position der Endoprothese liegen die Mittelpunkte 31, 32 zweckmäßig in der gleichen gemeinsamen Ebene 40. Somit wird in der gestreckten Position zuverlässig eine Arretierung gegen Rotationen um die zum Tibiaplateau 20 orthogonale Hochachse gewährleistet. Im Rahmen dieser Erfindung können ferner die Zylinderachsen 38, 39 alternativ auch in verschiedenen Ebenen angeordnet sein, welche einen vorgegebenen Winkel zu einander aufweisen. Desweiteren können in alternativen Ausgestaltungen die Mittelpunkte 31, 32 und/oder deren Verbindungslinie 34 außerhalb der gemeinsamen Ebene 40 angeordnet sein, um den jeweiligen Erfordernissen entsprechend die Arretierung bezüglich der erwähnten Hochachse und/oder die Freigabe zur Drehung wie einem natürlichen Kniegelenk zu ermöglichen. Desweiteren ist auf diese Weise eine gewünschte Überstreckung vorgegeben.

Aufgrund der zylindrischen Ausbildung der Tibiateile 11, 12 und der entsprechenden Lagerflächen der beiden Meniskuselemente können entsprechend dem Doppelpfeil 36 Bewegungen parallel zur Zylinderachse erfolgen. Des weiteren wird hierdurch ein Ausgleich bei operativ bedingten Abstandsänderungen der beiden Mittelpunkte 31, 32 gewährleistet. Auch bei unterschiedlichen Abständen wird aufgrund der Querbewegbarkeit und im übrigen unveränderten Anordnung des Tibiaplateaus und der beiden Tibiateile zuverlässig eine flächenhafte Anlage der einander korrespondierenden Lagerflächen gewährleistet, wobei Linienberührungen und hieraus resultierende hohe Flächenpressungen vermieden werden. Die zylindrischen Lagerflächen 13, 14 der Tibiateile 11, 12 weisen bezüglich der im Bereich der Tibia liegenden Drehachsen 38, 39 den Radius 43, 44 auf und komplementär hierzu sind die konvexen Lagerflächen der beiden Meniskuselemente 5, 6 ausgebildet. Obgleich in zweckmäßiger Weise die Zylinderradien 43, 44 gleich groß ausgebildet sind, können in einer hier nicht dargestellten alternativen Ausgestaltung diese Zylinderradien 43, 44 unterschiedlich groß ausgebildet sein.

Wie durch die strichpunktierten Linien 35 angedeutet, können die zylindrischen Lagerflächen bzw. die Zylinderachsen der Tibiateile 11, 12 zum Tibiaplateau 20 in einem Winkel 37 geneigt angeordnet sein. Die Zylinderachsen weisen somit einen Sturz gegen die vertikale Tibiaachse 33 auf. Für die beiden tibialen Lagerflächen können die Neigungswinkel bzw. der Sturz jeweils unterschiedlich groß ausgebildet sein. Ferner kann die Neigung der beiden tibialen Lagerflächen gegensinnig erfolgen. Durch die erläuterte einseitige oder beidseitige Kippung der Lagerflächen gegen das Tibiaplateau 20 können den Erfordernissen entsprechend wichtige Verbesserungen der Führungsfunktion und der biologischen Adaption erreicht werden. In allen Ausführungsformen liegen die Femurteile, die Meniskuselemente und die Tibiateile mit ihren korrespondierenden Lagerflächen großflächig aneinander. Die Meniskuselemente bestehen zweckmäßig aus dem gleichen Werkstoff wie Femurteile und Tibiateile. Es gelangen die in der Implantologie bewährten Werkstoffe, und zwar vor allem Keramik, sowohl für die Meniskuselemente als auch für Femurteil und Tibiateil zum Einsatz.

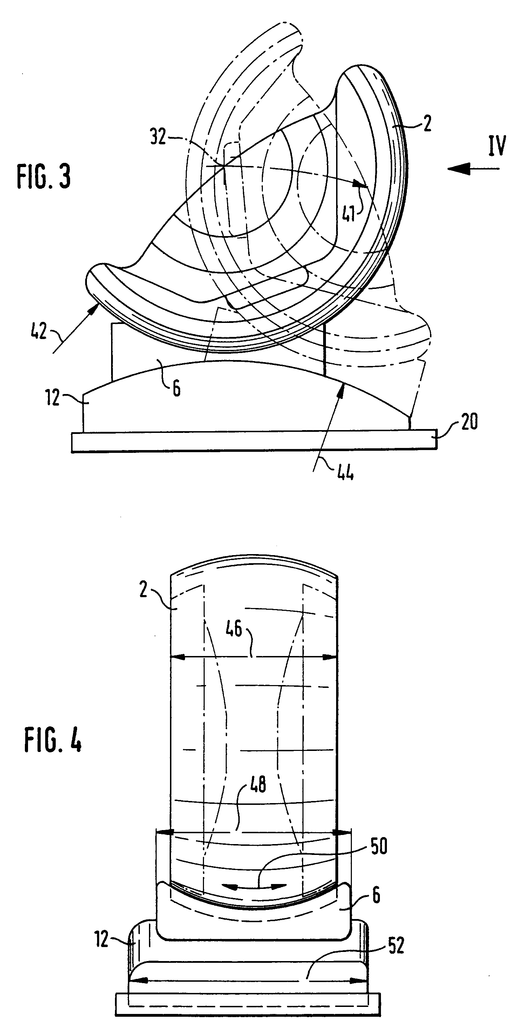

Fig. 3 zeigt das Femurteil 2 bei 5 Grad Überstreckung in ausgezogenen Linien. Hingegen ist mit strichpunktierten Linien die gebeugte Position des Femurteils sowie des Meniskuselements 6 angedeutet. Bei der Beugung durchläuft der Mittelpunkt 32 eine gekrümmte Bahn entsprechend der Linie 41. Der Kugelradius 42 des Femurteils 2 liegt im Bereich zwischen 25 bis 40 mm, während der Zylinderradius 44 des Tibiateiles 12 im Bereich zwischen 40 bis 60 mm vorgegeben werden kann. Wichtig ist insoweit, daß der Zylinderradius 44 um einen vorgegebenen Faktor größer ist, als der Kugelradius 42. Dieser Faktor liegt im Bereich zwischen 1,1 bis 2,5, zweckmäßig zwischen 1,2 und 2,0. Das Femurteil 2 erstreckt sich über einen Winkelbereich von etwa 180 Grad und weist im inneren eine gestufte Kontur auf.

In Fig. 4 ist mit strichpunktierten Linien die gestufte Innenkontur des Femurteils 2 dargestellt. Hierdurch ergibt sich bei stabiler Konstruktion eine Material- und Gewichtsersparnis und darüber hinaus die Möglichkeit einer zuverlässigen Verankerung im Femur. Die Breite 46 des Femurteils 2 ist kleiner als die Breite 48 des Meniskuselements 6. Diese Breitendifferenz wird in der Weise vorgegeben, daß bei den maximal zulässigen Drehwinkelbewegungen des Doppelpfeiles 50 die Lagerfläche 4 des Femurteils 2 vollständig in der konzentrischen Lagerfläche des Meniskuselements 6 auflagert. Die Breite 52 des Tibiateils 12 ist wiederum größer als die Breite 48 des Meniskuselements 6, um die bereits genannten Querbewegungen zu ermöglichen. Es versteht sich, daß Übergänge zwischen verschiedenen Flächenteilen abgerundet werden um scharfe Kanten zu vermeiden.

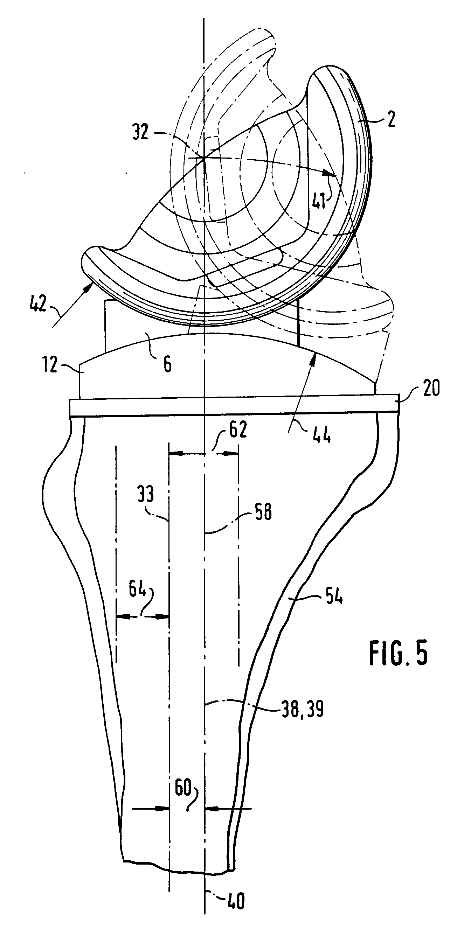

In Fig. 5 ist schematisch zusätzlich die Tibia 54 mit der vertikalen Tibiaachse 33 angedeutet. Die Zylinderachse 58 des Tibiateils 12 liegt nicht auf der Tibiaachse 33, sondern die Zylinderachse 58 ist dorsal in einem Abstand 60 verschoben angeordnet. Dieser Abstand 60 wird in einem Bereich 62 den Erfordernissen entsprechend vorgegeben, wobei dieser Bereich bis zu 10 mm groß sein kann. Gegebenenfalls kann die Zylinderachse 58 auch in einem Bereich 64 ventral zur Tibiachse 33 verschoben angeordnet sein. In der hier dargestellten besonderen Ausführung bei 5 Grad Überstreckung befindet sich der Mittelpunkt 32 der femuralen Lagerfläche ebenso wie die Zylinderachse 58 auf der gleichen vertikalen Achse, die in dem genannten Abstand zur Tibiaachse 33 parallel angeordnet ist.

### Bezugszeichenliste

- 1, 2: Femurteil
- 3, 4: Lagerfläche
- 5, 6: Meniskuselement
- 8: erste kugelförmige Lagerfläche von 6
- 10: zweite zylindrische Lagerfläche von 6
- 11, 12: Tibiateil
- 13, 14: zylindrisch konvexe Lagerfläche von 11, 12
- 16, 18: Begrenzungsteile
- 20: Tibiaplateau
- 22: Ansatz
- 24: Schneide
- 25: strichpunktierte Linie
- 26: Linie
- 27: Bohrung in 22
- 29: Befestigungsschraube
- 31, 32: Mittelpunkt von 1, 2
- 33: Tibiaachse
- 34: Verbindungslinie
- 35: Linie
- 36: Doppelpfeil
- 37: Winkel
- 38, 39: Zylinderachse
- 40: Ebene
- 41: gekrümmte Linie
- 42: Kugelradius
- 43, 44: Zylinderradius
- 46, 48: Breite
- 50: Doppelpfeil
- 52: Breite
- 54: Tibia
- 60: Abstand
- 62: Bereich

## Patentansprüche

1. Kniegelenk-Endoprothese mit einem Femurteil (2), einem Meniskuselement (6) und einem Tibiateil (12), wobei das Femurteil (2) und das Tibiateil (12) mit Lagerflächen an zugeordneten Lagerflächen (8, 10) des Meniskuselements (6) anliegen und wobei die Lagerfläche (4) des Femurteils (2) kugelförmig ausgebildet ist, welche an der kugelförmig konkaven Lagerfläche (8) des Meniskuselements (6) anliegt, dadurch gekennzeichnet, daß die Lagerfläche (14) des Tibiateils (12) zylindrisch konvex ausgebildet ist und an der koaxialen, zylindrisch konkav ausgebildeten Lagerfläche (10) des Meniskuselements (6) anliegt, wodurch das Meniskuselement (6) auf der zylindrischen Lagerfläche (14) zwangsgeführt und gegen Drehung um eine Achse gesichert ist, welche durch den Mittelpunkt (32) des Femurteils (2) und orthogonal zur Zylinderachse (39) der zylindrischen Lagerfläche (14) des Tibiateils (12) verläuft, und daß die genannte Zylinderachse (39) in lateral-medialer Richtung verläuft.

2. Kniegelenk-Endoprothese nach Anspruch 1, dadurch gekennzeichnet, daß das Meniskuselement (6) radial oder axial oder in Kombination davon zu der zylindrischen Lagerfläche (14) des Tibiateils (12) bewegbar ist, wobei das Meniskuslement (6) um die Zylinderachse (39) drehbar sowie translatorisch parallel zu dieser verschiebbar ist.

3. Kniegelenk-Endoprothese, nach Anspruch 1 oder 2, dadurch gekennzeichnet, daß auf einem Tibiaplateau (20) beabstandet zwei Tibiateile (11, 12) angeordnet sind, welche bevorzugt gleich große Radien der Lagerflächen (13, 14) aufweisen und/oder deren Radien (43, 44) bevorzugt zwischen 40 bis 60 mm groß sind.

4. Kniegelenk-Endoprothese nach einem der Ansprüche 1 bis 3, dadurch gekennzeichnet, daß die Zylinderachsen (38, 39) der Tibiateile (11, 12) im wesentlichen in der gleichen Ebene (40) angeordnet sind und daß in der gestreckten Position der Femurteile (1, 2) bezüglich der Tibiateile (11, 12) die Mittelpunkte (31, 32) und/oder eine zwischen diesen vorhandene Verbindungslinie (34) im wesentlichen parallel zu der, bevorzugt im wesentlichen innerhalb der gemeinsamen Ebene (40) angeordnet sind.

5. Kniegelenk-Endoprothese nach einem der Ansprüche 1 bis 4, dadurch gekennzeichnet, daß das Meniskuselement (5, 6) aus einem Werkstoff besteht, welcher im wesentlichen die gleiche Härte aufweist wie das Femurteil (1, 2) und/oder das Tibiateil (11, 12), wobei das Meniskuselement (5, 6) bevorzugt aus dem gleichen Werkstoff besteht wie das Femurteil (1, 2) und/oder das Tibiateil (11, 12).

6. Kniegelenk-Endoprothese nach einem der Ansprüche 3 bis 5, dadurch gekennzeichnet, daß das Tibiaplateau (20) auf seiner den Tibiateilen (11, 12) abgewandten Seite bevorzugt rohrförmige Ansätze (22) aufweist, innerhalb welcher konisch-selbsthemmende Bohrungen für Befestigungsschrauben vorgesehen sind, und/oder innerhalb welcher ein Gewinde eingebracht ist, um die Befestigungsschrauben gegen Lösen mit einem Gewindebolzen zu sichern.

7. Kniegelenk-Endoprothese nach einem der Ansprüche 1 bis 6, dadurch gekennzeichnet, daß der Radius (44) der insbesondere zylindrisch ausgebildeten Lagerfläche (11, 12) des Tibiateils um einen Faktor größer ist als der Kugelradius (42) des Femurteils (1, 2), wobei dieser Faktor im Bereich zwischen 1,1 bis 2,5 und bevorzugt zwischen 1,2 bis 2,0 liegt.

8. Kniegelenk-Endoprothese nach einem der Ansprüche 1 bis 7, dadurch gekennzeichnet, daß das Tibiateil (11, 12) an wenigstens einem Ende der Lagerfläche (14) ein Begrenzungsteil (16, 18) für die Bewegung des Meniskuselements (5, 6) aufweist.

9. Kniegelenk-Endoprothese nach einem der Ansprüche 3 bis 8, dadurch gekennzeichnet, daß die Zylinderachse der Tibiateile (11, 12) bezüglich des Tibiaplateaus (20) um einen Winkel (37) geneigt angeordnet ist und somit einen Sturz gegen die vertikale Tibiaachse (33) aufweist.

10. Kniegelenk-Endoprothese nach einem der Ansprüche 1 bis 9, dadurch gekennzeichnet, daß die Zylinderachse des Tibiateils (11, 12) gegenüber der vertikalen Tibiaachse (33) dorsal oder ventral in einem Abstand (60) verschoben angeordnet ist, und zwar in einem Bereich (62) bis zu 10 mm.

## Claims

1. Knee joint endoprosthesis comprising a femoral part (2), a meniscus element (6) and a tibial part (12), the femoral part (2) and the tibial part (12) bearing via bearing surfaces against associated bearing surfaces (8, 10) on the meniscus element (6) and the bearing surface (4) of the femoral part (2) being spherical and bearing against the spherically concave bearing surface (8) of the meniscus element (6), characterised in that the bearing surface (14) of the tibial part (12) is cylindrically convex and bears against the coaxial cylindrically concave bearing surface (10) of the meniscus element (6), as a result of which the meniscus element (6) is positively guided on the cylindrical bearing surface (14) and is secured against rotation about an axis extending through the centre point (32) of the femoral part (2) and orthogonally to the cylinder axis (39) of the cylindrical bearing surface (14) of the tibial part (12) and that the said cylinder axis (39) extends in the lateral-medial direction.

2. Knee joint endoprosthesis according to claim 1, characterised in that the meniscus element (6) is movable radially or axially or radially and axially in combination relative to the cylindrical bearing surface (14) of the tibial part (12), the meniscus element (6) being rotatable about the cylinder axis (39) and being displaceable in translation parallel thereto.

3. Knee joint endoprosthesis according to claim 1 or claim 2, characterised in that two tibial parts (11, 12) are arranged at a distance on a tibial plate (20), their bearing surfaces (13, 14) preferably having identical radii and/or their radii (43, 44) preferably being between 40 and 60 mm.

4. Knee joint endoprosthesis according to one of claims 1 to 3, characterised in that the cylinder axes (38, 39) of the tibial parts (11, 12) are arranged substantially in the same plane (40) and that when the femoral parts (1, 2) are in the extended position relative to the tibial parts (11, 12), the centre points (31, 32) and/or a connecting line (34) between them is/are arranged substantially parallel to and preferably substantially within the common plane (40).

5. Knee joint endoprosthesis according to one of claims 1 to 4, characterised in that the meniscus element (5, 6) consists of a material having substantially the same hardness as the femoral part (1, 2) and/or the tibial part (11, 12), the meniscus element (5, 6) preferably consisting of the same material as the femoral part (1, 2) and/or the tibial part (11, 12).

6. Knee joint endoprosthesis according to one of claims 3 to 5, characterised in that the tibial plate (20) is provided on its side remote from the tibial parts (11, 12) with preferably tubular projections (22) within which conically self-locking bores for fixing screws are provided and/or into which a thread is introduced in order to secure the fixing screws against loosening by means of a threaded bolt.

7. Knee joint endoprosthesis according to one of claims 1 to 6, characterised in that the radius (44) of the in particular cylindrical bearing surface (11, 12) of the tibial part is greater than the sphere radius (42) of the femoral part (1, 2) by a factor, this factor being in the range of between 1.1 and 2.5 and preferably between 1.2 and 2.0.

8. Knee joint endoprosthesis according to one of claims 1 to 7, characterised in that the tibial part (11, 12) is provided at least at one end of the bearing surface (14) with a part (16, 18) for limiting the movement of the meniscus element (5, 6).

9. Knee joint endoprosthesis according to one of claims 3 to 8, characterised in that the cylinder axis of the tibial parts (11, 12) is inclined at an angle (37) relative to the tibial plate (20) and thus slopes downwards relative to the vertical tibia axis (33).

10. Knee joint endoprosthesis according to one of claims 1 to 9, characterised in that the cylinder axis of the tibial part (11, 12) is arranged so that it is displaced dorsally or ventrally relative to the vertical tibia axis (33) at a distance (60), within a range (62) of up to 10 mm

## Revendications

1. Endoprothèse pour l'articulation du genou, comprenant une pièce fémorale (2), un élément de ménisque (6) et une pièce tibiale (12), la pièce fémorale (2) et la pièce tibiale (12) reposant avec des surfaces d'appui contre des surfaces d'appui associées (8, 10) de l'élément de ménisque (6) et la surface d'appui (4) de la pièce fémorale (2) étant conformée sphérique, laquelle repose contre la surface d'appui sphérique concave (8) de l'élément de ménisque (6), ***caracterisée en ce que*** la surface d'appui (14) de la pièce tibiale (12) est conformée cylindrique convexe et repose contre la surface d'appui concave cylindrique coaxiale (10) de l'élément de ménisque (6), de sorte que l'élément de ménisque (6) est guidé de manière forcée sur la surface d'appui cylindrique (14) et est immobilisé en rotation autour d'un axe passant par le centre (32) de la pièce fémorale (2) et orthogonal à l'axe (39) du cylindre de la surface d'appui cylindrique (14) de la pièce tibiale (12), et ***en ce que*** ledit axe (39) du cylindre s'étend dans la direction latérale-médiale.

2. Endoprothèse pour l'articulation du genou selon la Revendication 1, ***caractérisée en ce que*** l'élément de ménisque (6) est mobile dans le sens radial ou axial ou en combinaison par rapport à la surface d'appui cylindrique (14) de la pièce tibiale (12), l'élément de ménisque (6) pouvant tourner autour de l'axe (39) du cylindre et étant mobile en translation parallèlement à celui-ci.

3. Endoprothèse pour l'articulation du genou selon la Revendication 1 ou 2, ***caractérisée en ce que***, sur un plateau tibial (20), deux pièces tibiales (11, 12) sont placées à distance, lesquelles présentent de préférence des rayons identiques des surfaces d'appui (13, 14) et/ou dont les rayons (43, 44) sont de préférence compris entre 40 et 60 mm.

4. Endoprothèse pour l'articulation du genou selon l'une des Revendications 1 à 3, ***caractérisée en ce que*** les axes (38, 39) des cylindres des pièces tibiales (11, 12) sont situés pour l'essentiel dans le même plan (40) et ***en ce que***, dans la position étendue des pièces fémorales (1, 2) par rapport aux pièces tibiales (11, 12), les centres (31, 32) et/ou une ligne de jonction (34) présente entre ceux-ci sont situés pour l'essentiel parallèles, de préférence pour l'essentiel à l'intérieur, du plan commun (40).

5. Endoprothèse pour l'articulation du genou selon l'une des Revendications 1 à 4, ***caractérisée en ce que*** l'élément de ménisque (5, 6) est constitué d'un matériau qui présente pour l'essentiel la même dureté que la pièce fémorale (1, 2) et/ou la pièce tibiale (11, 12), l'élément de ménisque (5, 6) étant constitué de préférence du même matériau que la pièce fémorale (1, 2) et/ou la pièce tibiale (11, 12).

6. Endoprothèse pour l'articulation du genou selon l'une des Revendications 3 à 5, ***caractérisée en ce que*** le plateau tibial (20), sur son côté opposé aux pièces tibiales (11, 12), présente de préférence des embases tubulaires (22), à l'intérieur desquelles des perçages autobloquants coniques sont prévus pour des vis de fixation, et/ou à l'intérieur desquelles un filetage est ménagé, pour immobiliser les vis de fixation contre un dégagement, avec un goujon fileté.

7. Endoprothèse pour l'articulation du genou selon l'une des Revendications 1 à 6, ***caractérisée en ce que*** le rayon (44) de la surface d'appui (11, 12) de la pièce tibiale conformée en particulier cylindrique est plus grande d'un certain facteur que le rayon de sphère (42) de la pièce fémorale (1, 2), ce facteur étant compris entre 1,1 et 2,5, et de préférence entre 1,2 et 2,0.

8. Endoprothèse pour l'articulation du genou selon l'une des Revendications 1 à 7, ***caractérisée en ce que*** la pièce tibiale (11, 12) présente au moins à une extrémité de la surface d'appui (14) une partie (16, 18) de limitation du mouvement de l'élément de ménisque (5, 6).

9. Endoprothèse pour l'articulation du genou selon l'une des Revendications 3 à 8, ***caractérisée en ce que*** l'axe du cylindre des pièces tibiales (11, 12) est incliné d'un angle (37) par rapport au plateau tibial (20) et présente ainsi une déclivité par rapport à l'axe vertical (33) du tibia.

10. Endoprothèse pour l'articulation du genou selon l'une des Revendications 1 à 9, ***caractérisée en ce que*** l'axe du cylindre de la pièce tibiale (11, 12) est déplacé dans le sens dorsal ou ventral d'une distance (60) par rapport à l'axe vertical (33) du tibia, à savoir dans une zone (62) allant jusqu'à 10 mm.
